(19) Europäisches Patentamt
European Patent Office
Office européen des brevets

(11) Publication number: 0 002 835
A1

(12) # EUROPEAN PATENT APPLICATION

(21) Application number: 78101867.6

(22) Date of filing: 28.12.78

(51) Int. Cl.2: C 07 H 15/22, A 61 K 31/70

(30) Priority: 29.12.77 JP 158496/77

(43) Date of publication of application: 11.07.79
Bulletin 79/14

(84) Designated Contracting States: BE CH DE FR GB NL

(71) Applicant: Shionogi & Co., Ltd., 12, 3-chome Dosho-machi Higashi-ku, Osaka (JP)

(72) Inventor: Igarashi, Kikuo, 11-5, Nishinosotogawara Itami-shi, Hyogo (JP)
Inventor: Sugawara, Tamio, 22-1-12-103, Nyoidani Mino-shi, Osaka (JP)

(74) Representative: Vossius, Volker, Dr. et al, Vossius, Vossius, Hiltl, Tauchner, Heunemann Patentanwälte Siebertstrasse 4, D-8000 München 86 (DE)

(54) **Aminoglycoside derivatives, process for their manufacture and pharmaceutical compositions.**

(57) Aminoglycoside derivatives of the general formula (I)

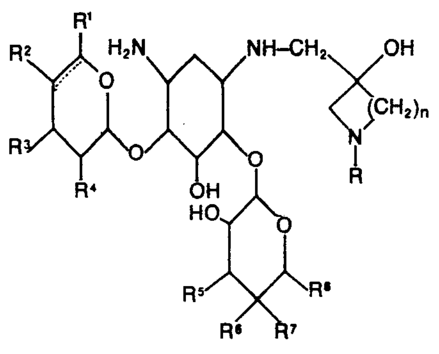

(I)

wherein

R is hydrogen, lower alkyl or aralkyl;
$R^1$ is aminomethyl, hydroxymethyl, 1-aminoethyl or 1-methylaminoethyl;
$R^2$, $R^3$, and $R^6$ are each hydrogen or hydroxy;
$R^4$ is hydroxy or amino;
$R^5$ is amino or methylamino;
$R^7$ is hydroxy or methyl;
$R^8$ is hydrogen or hydroxymethyl;
n is an integer of 1 to 3; and
the dotted line represents the presence or absence of a double bond,

and their salts with acids, a process for their manufacture and pharmaceutical compositions having antibiotic activity and containing compounds of the general formula (I).

EP 0 002 835 A1

VOSSIUS · VOSSIUS · HILTL · TAUCHNER · HEUNEMANN
PATENTANWÄLTE
MÜNCHEN

Our Ref.: M 938 EP
Case: F 3624 MIK

Shionogi & Co., Ltd.
Osaka, Japan

---

" Aminoglycoside Derivatives, Process for their Manufacture and Pharmaceutical Compositions "

---

Priority: 29 December 1977, Japan, No. 158 496

---

Aminoglycoside antibiotics, for example, streptomycins, kanamycins, gentamycins, tobramycins, etc. have been used in practice as broad spectrum antibiotics effective against gram-positive, gram-negative and acid-

fast bacteria. The aminoglycoside antibiotics, however, are sometimes accompanied by undesired side effects such as nephropathy and deafness. The occurrence of strains resistant to the aminoglycosides is another problem to be solved. It has been attempted to modify such aminoglycosides by acylating the 1-amino group with specific acyl groups in order to improve the antibiotic activity and relatively decrease the side effects.

It is an object of the invention to provide novel aminoglycoside derivatives having an excellent antibiotic action. More particularly, this invention relates to novel aminoglycoside antibiotic derivatives and their salts with acids represented by the general formula (I)

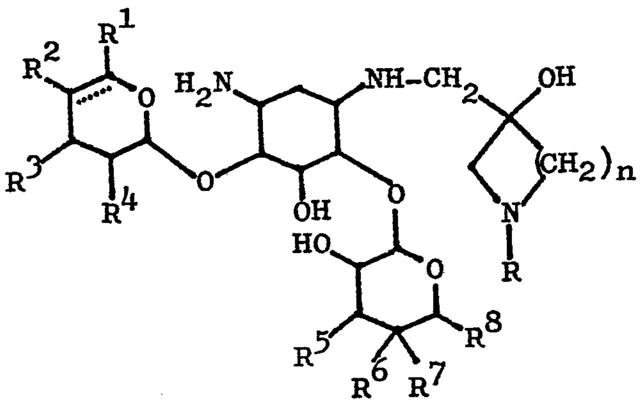

(I)

wherein R is hydrogen, lower alkyl or aralkyl ;

$R^1$ is aminomethyl, hydroxymethyl, 1-aminoethyl, or 1-methylaminoethyl ;

$R^2$, $R^3$, and $R^6$ are each hydrogen or hydroxy ;

$R^4$ is hydroxy or amino ;

$R^5$ is amino or methylamino ;

$R^7$ is hydroxy or methyl ;

$R^8$ is hydrogen or hydroxymethyl ;

n is an integer of 1 to 3 ; and

the dotted line represents the presence or absence of a double bond.

In the aforementioned general formula (I), the lower alkyl groups R denote $C_1$ to $C_5$ alkyl groups, for example a methyl, ethyl, propyl, isopropyl, butyl, isobutyl, s-butyl, t-butyl, pentyl, isopentyl, neopentyl, or t-pentyl group. Specific examples of aralkyl groups are the benzyl, phenethyl, and phenylpropyl group.

The novel aminoglycoside antibiotic derivatives (I) in this invention include the free bases and salts thereof, particularly non-toxic acid addition salts, for example salts with inorganic acids such as hydrochloric acid, hydrobromic acid, hydroiodic acid, sulfuric acid, phosphoric acid, carbonic acid, and the like, and salts with organic acids such as acetic acid, fumaric acid, malic acid, tartaric acid, maleic acid, citric acid, mandelic acid, ascorbic acid, gallic acid, and the like.

Typical examples of the aminoglycosides of the general formula (I) are:

1) 1-N-[(3-hydroxyazetidin-3-yl)methyl]tobramycin
2) 1-N-[(3-hydroxypyrrolidin-3-yl)methyl]tobramycin
3) 1-N-[(3-hydroxypiperidin-3-yl)methyl]tobramycin
4) 1-N-[(3-hydroxyazetidin-3-yl)methyl]kanamycin A
5) 1-N-[(3-hydroxypyrrolidin-3-yl)methyl]kanamycin A
6) 1-N-[(3-hydroxypiperidin-3-yl)methyl]kanamycin A.

The aminoglycosides used as starting materials in this invention containing a 2-deoxystreptamine moiety are represented by the general formula (II)

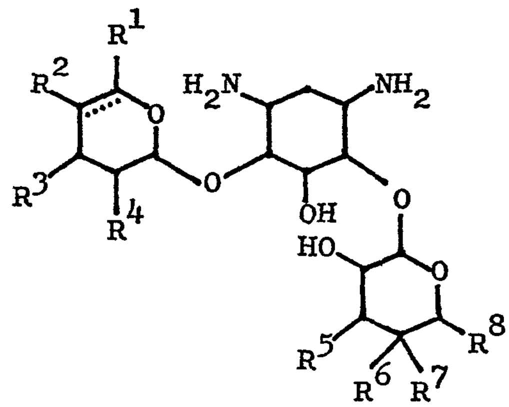

(II)

wherein $R^1$, $R^2$, $R^3$, $R^4$, $R^5$, $R^6$, $R^7$, $R^8$, and the dotted line each has the same meaning as mentioned above.

Representatives of the compounds (II) and their substituents are shown in Table I.

Table I

| Generic Name | $R^1$ | $R^2$ | $R^3$ | $R^4$ | $R^5$ | $R^6$ | $R^7$ | $R^8$ | dotted line |
|---|---|---|---|---|---|---|---|---|---|
| tobramycin | $CH_2NH_2$ | OH | H | $NH_2$ | $NH_2$ | H | OH | $CH_2OH$ | none |
| kanamycin A | " | " | OH | OH | " | " | " | " | " |
| " B | " | " | " | $NH_2$ | " | " | " | " | " |
| " C | $CH_2OH$ | " | " | " | " | " | " | " | " |
| deoxykanamycin A | $CH_2NH_2$ | " | H | OH | " | " | " | " | " |
| dideoxykanamycin B (dibekacin) | " | H | " | $NH_2$ | " | " | " | " | " |
| gentamicin $C_1$ | $CH(CH_3)NHCH_3$ | " | " | " | $NHCH_3$ | OH | $CH_3$ | H | " |
| " $C_2$ | $CH(CH_3)NH_2$ | " | " | " | " | " | " | " | " |
| " $C_{1a}$ | $CH_2NH_2$ | " | " | " | " | " | " | " | " |
| " B | " | " | OH | OH | " | " | " | " | " |
| sisomicin | " | " | H | $NH_2$ | " | " | " | " | double bond |

The aminoglycosides of the general formula (I) may be prepared by treatment of an aminoglycoside of the general formula (III

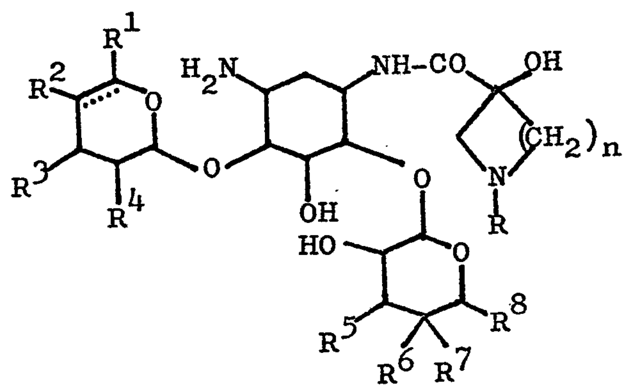

(III)

wherein R, $R^1$, $R^2$, $R^3$, $R^4$, $R^5$, $R^6$, $R^7$, $R^8$, n, and the dotted line each have the same meaning as mentioned above with a reducing agent. The aminoglycosides of the general formula (III) are described in Japanese Unexamined Patent Applications Nos. 51-130119, 52-112840, and 52-114176.

For example, one of the starting materials represented by the general formula (IIIa)

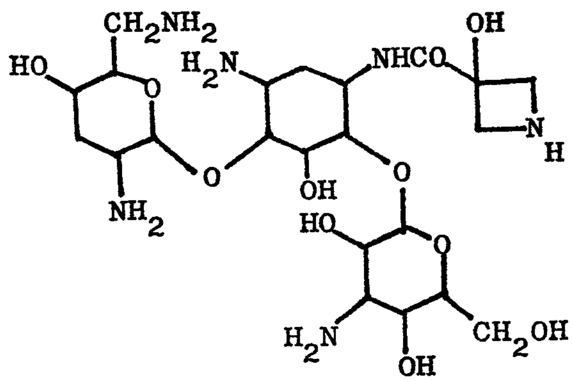

(IIIa)

may be prepared by acylation of tobramycin with the N-hydroxy-succinimido ester of 3-hydroxyazetidine-3-carboxylic acid,during which reaction the functional groups other than the 1-amino-group are properly protected. After termination of the acylation the functional groups are deprotected.

Representative reducing agents are diborane, lithium aluminium hydride, lithium aluminium hydride-aluminium chloride, sodium borohydride-aluminium chloride, sodium-borohydride-boron trifluoride etherate, and similar reducing agents which may usually be employed in selective reduction of the amide. The preferred reducing agent is diborane.

The reduction may be carried out in a suitable anhydrous solvent. In carrying out the reaction, an equimolar amount or excess amount of the reducing agent, preferably about 1 to 2 equivalents, may be used to 1 mole of the starting aminoglycoside of the general formula (III). This reaction proceeds well even at room temperature but, if required, it may be accelerated by carrying out the reaction under reflux of the reaction medium.

Preferably, this reaction is conducted under an inert gas atmosphere, more preferably under nitrogen. Representative solvents are ethers such as diethyl ether, dioxane, tetrahydrofuran, ethylene glycol mono- and dimethylether and their mixtures. The reaction is usually terminated within a period of several hours, in general 1 to 8 hours.

It is preferred that the starting aminoglycosides of the general formula (III) are employed in the form of their acid addition salts with strong acids such as trifluoroacetic acid because the salts are better soluble in the solvents.

The aminoglycoside derivatives (I) and their salts of the general formula (I) exhibit an excellent antibiotic activity. They are in general significantly more active against gram-positive and gram-negative bacteria than the corresponding unacylated aminoglycosides. The minimum inhibitory concentration (MIC, µg/ml) of the compounds of this invention and that of tolramycin are indicated in Table II.

Table II

| Bacteria \ Test compounds | (1) | (2) | (3) | (4) | (5) |
|---|---|---|---|---|---|
| Staphylococcus aureus S-25* | 50 | 25 | 25 | 12.5 | 100 |
| Staphylococcus epidermidis TB-302 | 3.13 | 1.56 | 3.13 | 1.56 | 3.13 |
| Escherichia coli EC-147* | 3.13 | 3.13 | 6.25 | 6.25 | 50 |
| Escherichia coli EC-148* | 3.13 | 3.13 | 12.5 | 6.25 | 100 |
| Escherichia coli W-677/JR 214* | 1.56 | 1.56 | 6.25 | 3.13 | 100 |
| Escherichia coli W-677/JR 225* | 1.56 | 0.78 | 3.13 | 3.13 | 50 |
| Escherichia coli W-677/JR-762* | 3.13 | 3.13 | 12.5 | 12.5 | >100 |
| Klebsiella pneumoniae K1-187* | 0.78 | 0.39 | 1.56 | 1.56 | 25 |
| Enterobacter cloacae C1-126* | 0.78 | 0.78 | 1.56 | 1.56 | 25 |
| Citrobacter freundii Ct-52* | 0.78 | 0.78 | 3.13 | 1.56 | 25 |
| Serratia marcescens MA-81* | 12.5 | 12.5 | 12.5 | 12.5 | >100 |
| Proteus mirabilis TB-617* | 1.56 | 1.56 | 6.25 | 3.13 | 6.25 |
| Proteus inconstans In-43* | 6.25 | 12.5 | 3.13 | 3.13 | 6.25 |
| Pseudomonas aeruginosa PP-6* | 0.78 | 0.78 | 3.13 | 3.13 | 50 |
| Pseudomonas aeruginosa TB-485* | 6.25 | 3.13 | 6.25 | 6.25 | 25 |

(Note) (1) = 1-N-[(3-hydroxyazetidin-3-yl)methyl]tobramycin

(2) = 1-N-[(3-hydroxypyrrolidin-3-yl)methyl]tobramycin

(3) = 1-N-[(3-hydroxyazetidin-3-yl)methyl]kanamycin A

(4) = 1-N-[(3-hydroxypyrrolidin-3-yl)methyl]kanamycin A

(5) = tobramycin

* = tobramycin-resistant strains

As can be seen from Table II, the compounds (I) of this invention are valuable antibiotics effective against various species of gram-positive and gram-negative bacteria, and useful as drugs for administration to humans and animals. They can be used for preventing or treating infectious deseases caused by aminoglycoside-sensitive strains and resistant strains (e.g. Escherichia coli, Klebsiella pneumoniae, Proteus mirabilis, Pseudomonas aeruginosa).

The compounds (I) of this invention can also be used as disinfectants to prevent the growth of bacteria living in perishables, feedstuffs or sanitary articles.

The compounds (I) of this invention can be confected in a wide variety of oral or parenteral dosage forms, either alone or in admixture with other co-acting substances. The pharmaceutical compositions may consist of a mixture of 0.01 to 99 % of the compounds (I) and a pharmaceutical carrier or carriers which can be solids or liquids in which the compounds (I) can be dissolved, dispersed or suspended. They can be prepared in a unit dosage form. The solid compositions can be produced as tablets, powder, dry syrups, troches, granules, capsules, pills, suppositories or similar solid preparations. The liquid compositions can be prepared in the form of injectable preparations, ointments, dispersions, inhalants, suspensions, solutions, emulsions, syrups, or elixiers. Any diluents (e.g.starch, sucrose, lactose, calcium carbonate, kaolin), bulking agents (e.g.lactose, sugar, salt, glycine, starch, calcium carbonate, calcium phosphate, kaolin, bentonite, talc, sorbitol), binders (e.g. starch, acacia, gelatin, glucose, sodium alginate, tragacanth, carboxymethylcellulose, sorbitol, polyvinyl-pyrrolidone), disintegrating agents (e.g. starch, agar, carbonate sodium laurylsulfate), lubricants (e.g. stearic acid, talc, paraffin, boric acid, silica, sodium benzoate, polyethylene-glycol, cacao oil, magnesium sulfate), emulsifying agents (e.g. lecithin, sorbitan

monooleate, acacia), suspending agents (e.g. sorbitol, methylcellulose, glucose, sugar syrup, gelatin, hydroxyethylcellulose, carboxymethylcellulose, aluminium stearate gel, hydrogenated fats), solvents (e.g. water, peanut oil, sesame oil, methyl oleate), preservatives (e.g. methyl or ethyl p-hydroxybenzoate, sorbic acid), edible coloring agents, aromatic substances, solubilizing agents, buffers, stabilizing agents, dispersing agents, wetting agents, antioxidants, and the like can be used in the conventional manner as far as they do not have an adverse effect on the compounds(I).

The compounds (I) of this invention, particularly their sulfates, are readily soluble in water and conveniently used as a solution for intravenous, intramuscular or subcutaneous injection. The compounds (I) can be dissolved in an aqueous or oily solvent for injection to give an injectable solution in an ampoule; in order to preserve the injectable preparation for a long period of time, it is appropriate to make a vial preparation containing the compound (I) in a crystalline form, as a powder, or in a microcrystalline form, or as a lyophilizate. The vial preparation may be dissolved or suspended in the said solvent for injection immediately before use. The preparation may contain the said preservatives.

Further, the compounds (I) of this invention can be used as suppositories, ointments for topical use or in ophtalmology powders for topical use and similar preparations preparable according to well-known methods. The external preparation can contain 0.01 to 99 % of a compound (I) of this invention, together with a necessary amount of a pharmaceutical carrier and/or diluent and/or auxiliary agent as stated above.

In order to treat or prevent infections caused by bacteria in humans or domestic animals the compounds (I) of this invention are administered as a single or divided dose of 0.01 to 5 g/kg a day if injected,of 0.01 to 10 g/kg a day if administered orally,or of 0.01 to 10 g/kg a day if applied locally at intervals of 3 to 12 hours.

The method can be used to treat or prevent some infectious diseases caused by bacteria sensitive to the compounds of this invention, e.g. staphylodermia, anthropozoonosis, cystitis, pyelitis, pneumonia, pneumonitis, bronchitis empyema, naspharyngitis, tonsilitis, rhinitis, dermatitis, pustulosis, abscesses, wounds and soft tissue infections, ear infections, osteomyelitis, septicemia, enteritis, urinary tract infections, and pyelonephritis.

Preferably, the compounds (I) of this invention are given to a patient in the form of a pharmaceutical preparation, e.g. powder, dry syrup, tablets, troches, granules, capsules, pills, suppositories, injections, ointments, dispersions, inhalants, suspensions, solutions, emulsions, syrups and elixirs. They may be preserved in a unit dosage form, e.g. tablets, troches, capsules, injectable preparations, vials, granules or powders in a separte container of a package.

The following example is provided to further illustrate this invention.

Example

Preparation of 1-N/(3-hydroxyazetidin-3-yl)methyl/tobramycin sulfate

500 mg 1-N-/(3-hydroxyazetidin-3-yl)carbonyl/ tobramycin are dissolved in 4 ml of anhydrous trifluoracetic acid under ice-cooling and the excess amount of trifluoroacetic acid is evaporated under reduced pressure. The resulting vitrified residue is dissolved in 17 ml of dry tetrahydrofuran and 66 ml of a 1M solution of diborane in tetrahydrofuran added dropwise thereto over a period of 4 hours under a nitrogen atmosphere. The reaction mixture is stirred at 50°C for 6 hours, cooled, and after decomposition of the excess amount of diborane with a small amount of water, evaporated under reduced pressure. The residue is dissolved in 30 ml of water, adjusted to pH 11 to 12 with an aqueous sodium hydroxide solution, and then adjusted to pH 5 with 10 % hydrochloric acid. The solution is adsorbed on a column of 100 ml of Amberlite CG-50 ($NH_4^+$ type) and after washing of the column with 200 ml of water, eluted with 1 L of water and 1 L of 1.5N aqueous ammonium hydroxide solution by the gradient method. The eluate is collected in fractions of 12 ml. Fraction Nos. 74 to 79 are combined and evaporated under reduced pressure to yield 229 mg of 1-N-/(3-hydroxyazetidin-3-yl)methyl/-tobramycin in 47 % yield.

A solution of 140 mg of the above product in 20 ml of water is adjusted to pH 6.0 with about 5 ml of 0.11N sulfuric acid, concentrated to about 1 to 2 ml under reduced pressure, and treated with 60 ml of ethanol. The resulting precipitate is collected by filtration, washed with ethanol, dissolved in water, treated with active carbon (Norit A), and filtered through a pyrex glass filter. The filtrate is lyophilized, and the lyophilizate is allowed to stand to absorb the moisture up to stationary state yielding 166 mg of 1-N-/(3-hydroxyazetidin-3-yl)methyl/tobramycin sulfate.

$[\alpha]_D^{23.5}$ + 62.5 ± 1.0° (c=1.036, $H_2O$)

Elemental Analysis

Calcd(%) for $C_{22}H_{44}N_6O_{10}\cdot 2.3H_2SO_4\cdot 7.5H_2O$ :

C,28.43 ; H,6.90 ; N,9.04 ; S,7.94.

Found(%) :

C,28.43 ; H,6.62 ; N, 8.97 ; S,7.86.

NMR : $\delta_{ppm}^{D_2O}$ 6.30(d, J=4Hz), 5.68(d, J=3.5Hz).

Compounds indicated in Table III may be prepared in the same manner.

Table III

| Ex. No. | Compounds | Yield (%) | Optical Rotation | Elemental Analysis | NMR : $\delta^{D_2O}$ |
|---|---|---|---|---|---|
| 2 | 1-N-[(3-hydroxypyrrolidin-3-yl)methyl]tobramycin sulfate | 45 | $[\alpha]_D^{23.5}$ = +61.2±1.0° (c=1.017, $H_2O$) | ($C_{23}H_{46}N_6O_{10}\cdot 2.3H_2SO_4\cdot 9.5H_2O$) Calcd(%) : C,28.67 ; H,7.28 ; N, 8.72 ; S,7.66. Found(%) : C,28.85 ; H,6.98 ; N, 8.75 ; S,7.41. | 6.36(d, J=3.5Hz), 5.68(d, J=3Hz) |
| 3 | 1-N-[(3-hydroxyazetidin-3-yl)methyl]kanamycin A sulfate | 39 | $[\alpha]_D^{24.0}$ = +59.9±1.0° (c=1.000, $H_2O$) | ($C_{22}H_{43}N_5O_{12}\cdot 2H_2SO_4\cdot 9H_2O$) Calcd(%) : C,28.47 ; H,6.84 ; N, 7.55 ; S,6.91. Found(%) : C,28.25 ; H,6.62 ; N, 7.41 ; S,7.21. | 6.15(d, J=3.5Hz), 5.70(d, J=3Hz). |
| 4 | 1-N-[(3-hydroxypyrrolidin-3-yl)methyl]kanamycin A sulfate | 39 | $[\alpha]_D^{24.0}$ = +54.0±0.9° (c=1.010, $H_2O$) | ($C_{23}H_{45}N_5O_{12}\cdot 2H_2SO_4\cdot 10H_2O$) Calcd(%) : C,28.78 ; H,7.25 ; N, 7.30 ; S,6.68. Found(%) : C,28.49 ; H,6.95 ; N, 7.17 ; S,6.92. | 6.13(d, J=3.5Hz), 5.73(d, J=3Hz) |

What is claimed is:

1. Aminoglycoside derivatives of the general formula (I)

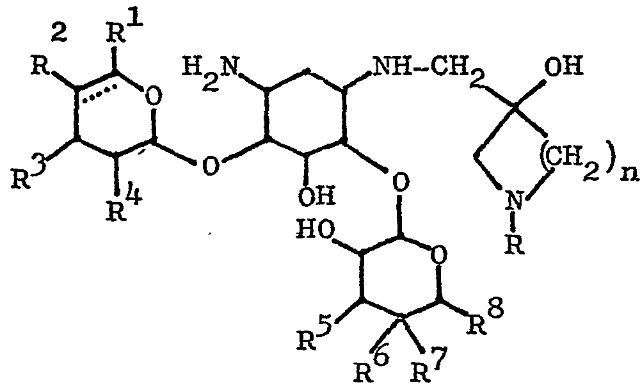

(I)

wherein R is hydrogen, lower alkyl or aralkyl ;

$R^1$ is aminomethyl, hydroxymethyl, 1-aminoethyl or 1-methylaminoethyl ;

$R^2$, $R^3$, and $R^6$ are each hydrogen or hydroxy ;

$R^4$ is hydroxy or amino ;

$R^5$ is amino or methylamino ;

$R^7$ is hydroxy or methyl ;

$R^8$ is hydrogen or hydroxymethyl ;

n is an integer of 1 to 3 ; and

the dotted line represents the presence or absence of a double bond,

and their salts with acids.

2. A compound claimed in claim 1, namely 1-N-[(3-hydroxyazetidin-3-yl)methyl]tobramycin.

3. A compound claimed in claim 1, namely 1-N-[(3-hydroxypyrrolidin-3-yl)methyl]tobramycin.

4. A compound claimed in claim 1, namely 1-N-[(3-hydroxypiperidin-3-yl)methyl]tobramycin.

5. A compound claimed in claim 1, namely 1-N-[(3-hydroxyazetidin-3-yl)methyl]kanamycin A.

6. A compound claimed in claim 1, namely 1-N-[(3-hydroxypyrrolidin-3-yl)methyl]kanamycin A.

7. A compound claimed in claim 1, namely 1-N-[(3-hydroxypiperidin-3-yl)methyl]kanamycin A.

8. A process for preparing the compounds claimed in claim 1, which comprises reducing an aminoglycoside of the general formula (II)

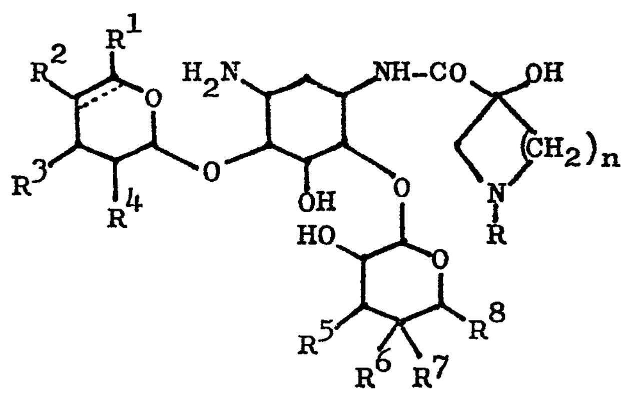

(II)

wherein R is hydrogen, lower alkyl or aralkyl ;

$R^1$ is aminomethyl, hydroxymethyl, 1-aminoethyl or 1-methylaminoethyl ;

$R^2$, $R^3$, and $R^6$ each is hydrogen or hydroxy ;

$R^4$ is hydroxy or amino ;

$R^5$ is amino or methylamino ;

$R^7$ is hydroxy or methyl ;

$R^8$ is hydrogen or hydroxymethyl ;
n is an integer of 1 to 3; and
the dotted line represents the presence or absence of a double bond,

the functional groups other than the 1-amino-group being optionally protected, followed if necessary by deprotection of the functional groups, and if desired converting the aminoglycoside derivative obtained into its salt by treatment with an inorganic or organic acid.

9. A process claimed in claim 8, where the reducing agent is diborane, lithium aluminium hydride, lithium aluminium hydride-aluminium chloride, sodium borohydride-aluminium chloride or sodium borohydride-boron trifluoride etherate.

10. Pharmaceutical compositions comprising an effective amount of a compound claimed in claim 1 and a pharmaceutically acceptable carrier and/or diluent and/or auxiliary agent.

European Patent Office

**EUROPEAN SEARCH REPORT**

0002835

Application number
EP 78 10 1867

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.²) |
| | FR - A - 2 240 015 (SHERICO)<br>* Pages 116-126 *<br>-- | 1,10 | C 07 H 15/22<br>A 61 K 31/70 |
| P | FR - A - 2 369 295 (SHIONOGI)<br>* Pages 26-29 *<br>---- | 1,8,10 | |
| | | | TECHNICAL FIELDS SEARCHED (Int.Cl.²)<br>C 07 H 15/22<br>A 61 K 31/70 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 22-03-1979 | VERHULST |

EPO Form 1503.1 06.78